(19) Europäisches Patentamt
European Patent Office
Office européen des brevets

(11) EP 1 405 633 A1

(12) **DEMANDE DE BREVET EUROPEEN**

(43) Date de publication:
**07.04.2004 Bulletin 2004/15**

(51) Int Cl.7: **A61K 7/48**

(21) Numéro de dépôt: **03292265.0**

(22) Date de dépôt: **15.09.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **03.10.2002 FR 0212261**

(71) Demandeur: **L'OREAL**
**75008 Paris (FR)**

(72) Inventeurs:
- **Boulle, Christophe**
**Lagny S/Marne 77400 (FR)**
- **Dalko, Maria**
**91190 Gif S/Yvette (FR)**
- **Cavezza, Alexandre**
**93290 Tremblay-en-France (FR)**

(74) Mandataire: **Renard, Emmanuelle**
**L'OREAL - D.I.P.I.**
**25-29 Quai Aulagnier**
**92600 Asnières (FR)**

(54) **Composition, notamment cosmétique, comprenant une aminecétone aromatique**

(57) La présente invention concerne un procédé de traitement cosmétique d'une peau ridée, comprenant l'application topique sur ladite peau d'une composition, adaptée à une application topique sur la peau, comprenant, dans un milieu physiologiquement acceptable, au moins une amine secondaire ou tertiaire carbonylée de formule (I) :

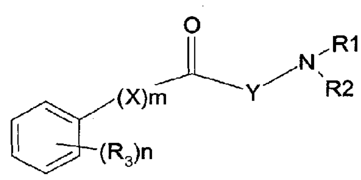

(I)

Elle concerne également une nouvelle famille d'amines carbonylées, ainsi que les compositions cosmétiques les contenant.

EP 1 405 633 A1

## Description

La présente invention concerne un procédé de traitement cosmétique d'une peau ridée, comprenant l'application topique sur ladite peau d'une composition, adaptée à une application topique sur la peau, comprenant, dans un milieu physiologiquement acceptable, au moins une amine secondaire ou tertiaire carbonylée de formule donnée. Elle concerne également une nouvelle famille d'amines carbonylées, ainsi que les compositions cosmétiques les contenant.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse, ou en prévenant la dégradation, des fibres élastiques qui composent le tissu cutané.

Bien que ces traitements permettent d'agir sur les rides et ridules dues au vieillissement chronologique ou intrinsèque, ainsi que sur celles dues au photo-vieillissement, ils n'ont pas d'effet sur les rides et ridules d'expression, lesquelles nécessitent une intervention sur la composante contractile musculaire des rides présente dans la peau.

Jusqu'à présent, le seul moyen couramment utilisé pour agir sur les rides d'expression est la toxine botulique qui est notamment injectée dans les rides de la glabelle qui sont les rides inter-sourcillières (voir J.D. Carruters et al., J. Dermatol. Surg. Oncol., 1992, 18, pp. 17-21).

La Demanderesse a en outre proposé divers composés susceptibles d'offrir un effet dermo-décontractant lorsqu'ils sont appliqués topiquement sur la peau, permettant ainsi d'agir par une autre voie sur les rides d'expression. Parmi ces composés, on peut notamment citer les antagonistes des récepteurs associés aux canaux calciques (FR-2 793 681 ), et en particulier le manganèse et ses sels (FR-2 809 005) et l'alvérine (FR-2 798 590) ; et les agonistes des récepteurs associés aux canaux chlore, dont la glycine (EP-0 704 210) et certains extraits *d'Iris pallida* (FR-2 746 641).

Il reste toutefois le besoin de disposer de composés efficaces pour lisser ou estomper les rides et ridules d'expression.

Or, la Demanderesse a découvert avec étonnement que certaines amines secondaires et tertiaires permettaient de satisfaire ce besoin.

On connaît, certes, du document EP-1 090 630 certaines amines secondaires et tertiaires ayant la propriété d'augmenter la synthèse de collagène par les fibroblastes et d'hydrater la peau, utiles contre la peau sèche et la dermatite atopique, et qui ont également une efficacité sur les rides. Toutefois, les amines carbonylées citées dans ce document ne comportent pas de groupe phényle et sont telles que le groupe carbonyle est directement adjacent à l'atome d'azote. En outre, elles n'ont pas d'effet sur les rides et ridules d'expression.

On connaît par ailleurs du document WO 93/05763 certaines amines di- et trisubstituées par au moins deux chaînes portant chacune au moins un groupe hydroxy. Ces amines augmentent la différenciation des kératinocytes, limitent l'épaississement UV-induit de l'épiderme et sont utiles pour prévenir et traiter les rides induites par le rayonnement UVB. Il n'est pas suggéré que ces amines, différentes de celles objet de la présente invention en ce sens qu'elles ne comprennent pas de groupement carbonyle, aient un quelconque effet sur les rides et ridules d'expression.

De manière analogue, le document EP-0 691 327 divulgue une famille très vaste d'amines mono-, di- ou trisubstituées décrites comme efficaces pour lisser les rides. Les amines exemplifiées dans cette demande de brevet ne sont pas substituées par des chaînes susceptibles de comprendre un groupement carbonyle, contrairement aux amines objet de la présente invention. En outre, il n'est pas suggéré qu'elles aient un quelconque effet sur les rides et ridules d'expression.

D'autres amines carbonylées encore ont été décrites dans le document US-6,372,772. Ces composés se distinguent de ceux objet de la présente invention en ce sens que la chaîne reliant le groupement carbonyle à l'atome d'azote comporte un substituant méthylène. Ce groupe méthylène, conjugué avec le groupe carbonyle et positionné en tant que substituant de la chaîne, constitue un bon accepteur de Michaël, qui est capable de capter des radicaux et les nucléophiles en général. Il confère à la molécule une photo-réactivité qui n'est pas souhaitable pour des applications cosmétiques dans la mesure où elle peut générer des problèmes d'innocuité.

La Demanderesse a maintenant découvert qu'en sélectionnant certaines amines secondaires et tertiaires carbonylées de structure simple, il était possible d'obtenir des compositions cosmétiques efficaces pour lisser les rides et ridules d'expression.

Certaines de ces amines ont déjà été décrites, par exemple dans la publication de Halise Inci Gul et al., Antifungical Evaluation of bis Mannich Bases derived from Acetophenones and their Corresponding Piperidinols and Stability Studies, Biological and Pharmaceutical Bulletin, Vol. 25, No. 10, pp. 1307-1310 (2002), comme antifongiques

utilisables contre les dermatophytes.

D'autres amines comprises dans la structure générale des amines selon la présente invention ont été décrites comme relaxants musculaires par action sur le système nerveux central (WO 95/18092 et Chawla H. P. S. et al., Agents acting on the Central Nervous System, XII. 3-Tert-Aminopropiophenones as central muscle relaxants and diuretics", Journal of Medicinal Chemistry, 13(3), 480-8 (1970)). Il n'était toutefois pas prévisible que ces composés aient une action de relaxation sur des fibroblastes permettant d'envisager leur utilisation topique sur la peau en vue de réduire les rides d'expression.

Il a, certes, été décrit précédemment par la Demanderesse l'utilisation de l'alvérine, qui est une amine trisubstituée, comme agent dermo-décontractant destiné à lisser les rides d'expression. Toutefois, à la différence des composés objet de la présente invention, l'alvérine ne renferme pas de groupement carbonyle. Or, il n'était pas évident que l'activité dermo-décontractante de l'alvérine soit conservée et même améliorée par introduction de groupements carbonyle dans sa molécule.

La présente invention a donc pour objet un procédé de traitement cosmétique d'une peau ridée, comprenant l'application topique sur ladite peau d'une composition, adaptée à une application topique sur la peau, comprenant, dans un milieu physiologiquement acceptable, au moins composé de formule (I) :

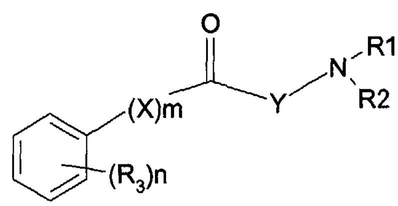

(I)

dans laquelle :

$R_1$ désigne un atome d'hydrogène ou un groupe alkyle linéaire ou ramifié, saturé ou insaturé, en $C_1$-$C_8$,
$R_2$ désigne :

- un groupe alkyle linéaire, ramifié ou cyclique, saturé ou insaturé, en $C_1$-$C_{20}$, éventuellement substitué par un groupe =O, et/ou par un plusieurs groupes -CN, -OR, -SR, -NRR', -COR, -COOR, -CONRR', -NR-CO-R', -NR-CO-NR'R", $-CF_3$ et/ou par un ou plusieurs atomes d'halogène et/ou par un groupe phényle éventuellement substitué par un groupe alkyle en $C_1$-$C_4$, un groupe alcoxy en $C_1$-$C_4$, un groupe hydroxy, un atome d'halogène et/ou un groupe $CF_3$,
- un groupe aryle éventuellement substitué par au moins un groupe choisi parmi : un groupe alkyle en $C_1$-$C_4$, un groupe alcoxy en $C_1$-$C_4$, un groupe hydroxy, un atome d'halogène ou un groupe $CF_3$,, ou
- un hétérocycle,

où R, R' et R" désignent indépendamment un atome d'hydrogène ou un groupe alkyle en $C_1$-$C_4$, linéaire ou ramifié, ou un groupe aryle,

$R_3$ désigne un groupe alkyle linéaire, ramifié ou cyclique, saturé ou insaturé, en $C_1$-$C_{12}$, ou un groupe aryle, éventuellement substitués par un groupe hydroxy, un groupe alcoxy en $C_1$-$C_4$ ou un atome d'hydrogène, un groupe -CN, -OR, -SR, -NRR', -COR,-COOR, -CONRR', -NR-CO-R', -NR-CO-NR'R" ou $-CF_3$ ou un atome d'halogène,

où R, R' et R" ont la signification indiquée précédemment,

X est un groupe alkylène linéaire en $C_1$-$C_9$, saturé ou insaturé, éventuellement substitué,
Y est un groupe alkylène linéaire en $C_1$-$C_{10}$, saturé ou insaturé, éventuellement substitué,

les substituants de X et Y étant indépendamment choisis parmi les groupements -OR, -SR, -NRR', -COR, -COOR, -CO-NRR', -NR-CO-R'R" et $CF_3$ où R, R' et R" ont la signification donnée ci-dessus,

m est 0 ou 1,
n est compris entre 0 et 3,

ou son sel d'addition avec un acide.

Dans la formule (I), les groupes alkyle peuvent être choisis, selon le cas, parmi les groupes : méthyle, éthyle, n-propyle, isopropyle, n-butyle, isobutyle, tert-butyle, pentyle, hexyle, heptyle, octyle, nonyle, décyle, undécyle, dodécyle, myristyle, palmityle, stéaryle et arachidyle.

De son côté, le groupe aryle est de préférence un groupe phényle.

Les groupes alkylène peuvent être des groupes méthylène, éthylène, propylène, butylène, pentylène, hexylène, heptylène, octylène, nonylène voire décylène.

L'atome d'halogène peut être un atome de fluor, de chlore, de brome ou d'iode.

Comme hétérocycles, on peut notamment citer les hétérocycles saturés ou insaturés, contenant de 1 à 3 hétéroatomes choisis parmi l'oxygène, l'azote et le soufre.

Comme sels du composé de formule (I), on peut citer les sels obtenus par addition du composé de formule (I) avec un acide inorganique, choisi notamment parmi les acides chlorhydrique, sulfurique, nitrique et phosphorique, ou avec un acide organique, choisi en particulier parmi les acides succinique, fumarique, lactique, glycolique, citrique et tartrique.

Les composés de formule (I) peuvent notamment être préparés comme décrit dans BADOSOV. E. P. et al, Chemistry of β-Amino Ketones, VII. Synthesis of Substituted Methyl and Phenyl β-[N-methyl-N(β-acetylethyl)]aminoethyl ketones by aminomethylation of ketones with formaldehyde and the salts of methyl and phenyl β-methylaminoalkyl ketones, Zhurnal Organicheskoi Khimii, Vol. 11, No. 5, pp. 972-977, Mai 1975. La synthèse de ces composés a par ailleurs été décrite dans VON K.

THIELE et al., Neue Piperidinderivative aus herzwirksamen -Aminoketonen, Arzneim. Forsch., 18(10), 1263-9 (1968).

Selon une forme d'exécution préférée de l'invention, le composé de formule (I) est tel que l'une au moins des conditions suivantes, et de préférence toutes ces conditions, sont satisfaites :

- m =0
- n=0
- Y est un groupe alkylène linéaire en $C_1$-$C_3$,
- $R_1$ est un groupe alkyle en $C_1$-$C_3$,
- $R_2$ est un groupe alkyle en $C_1$-$C_3$ substitué par un groupe arylcarbonyle

dans la formule (I), ou son sel avec un acide inorganique.

De façon plus préférentielle encore, le composé de formule (I) est tel que :

- m = 0
- n = 0
- Y est un groupe éthylène,
- $R_1$ est un groupe méthyle, et
- $R_2$ est un groupe éthyle substitué par un groupe benzoyle

dans la formule (I), ou son sel avec l'acide chlorhydrique.

Un tel composé, qui répond à la formule (Ia) ci-dessous :

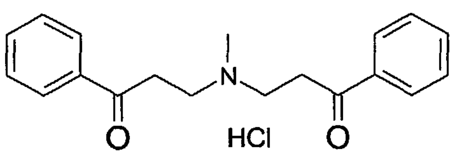

(Ia)

est notamment disponible auprès de la société SALOR sous la référence commerciale S35,861-4. Il peut en variante être préparé par aminométhylation d'acétophénone à l'aide du chlorhydrate de 3-méthylamino-1-phenyl-1-propanone (obtenu lui-même par réaction de la méthylamine sur la phenyl isopropényl cétone) et de formaldéhyde, comme décrit dans la première publication indiquée plus haut.

D'autres exemples de composés de formule (I) utiles dans la présente invention comprennent les composés (Ib) à (Ii) suivants :

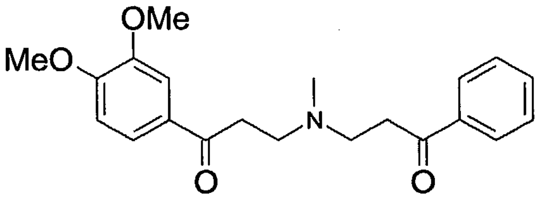

**(Ib)**

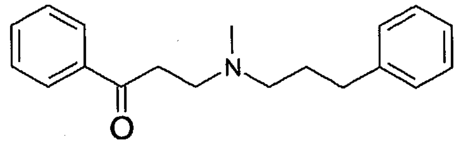

**(Ic)**

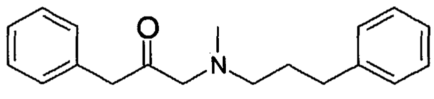

**(Id)**

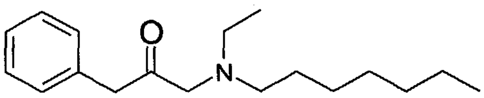

**(Ie)**

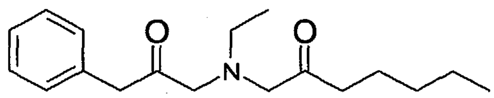

**(If)**

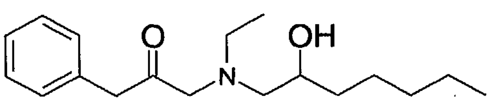

**(Ig)**

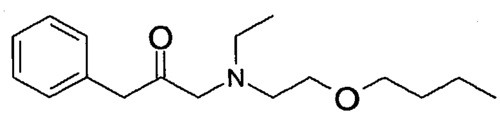

**(Ih)**

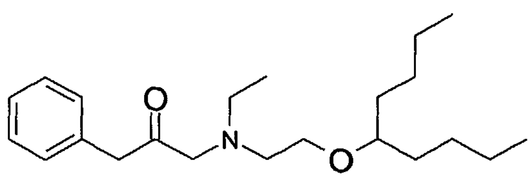

**(Ii)**

Comme cela sera démontré dans les Exemples ci-après, la Demanderesse a mis en évidence un effet dermo-décontractant des composés de formule (I) selon l'invention, qui permet d'envisager leur utilisation, plus particulièrement dans le lissage des rides et ridules d'expression.

L'invention a donc aussi pour objet l'utilisation cosmétique d'au moins un composé de formule (I) tel que défini ci-dessus, dans une composition adaptée à une application topique sur la peau, comme agent destiné à lisser les rides et ridules, en particulier d'expression.

La Demanderesse a par ailleurs mis en évidence que certains des composés de formule (I) étaient nouveaux et présentaient une activité dermo-décontractante intéressante.

L'invention a donc également pour objet une sous-famille de composés de formule (I), qui répondent à la formule (II) ci-dessous :

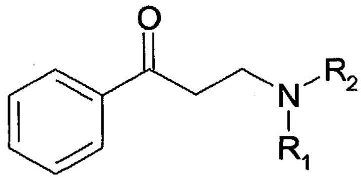

(II)

dans laquelle :

$R_1$ est un atome d'hydrogène ou un groupe méthyle ou éthyle,
$R_2$ est un radical alkyle linéaire ou ramifié en $C_6$-$C_{20}$ pouvant être substitué par un groupe oxo ou un groupe hydroxyle, ainsi que leurs sels d'addition avec un acide.

L'acide utilisé pour salifier les composés de formule (II) peut être tout acide physiologiquement acceptable, tel que défini précédemment.
Des exemples de composés de formule (II) répondent aux formules (IIa) à (IId) suivantes :

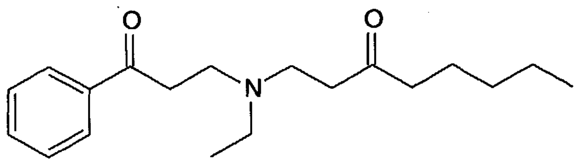

(IIa)

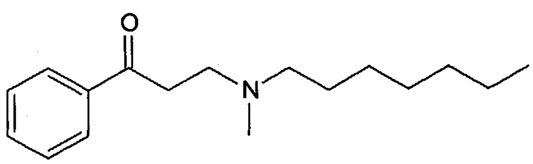

(IIb)

HCl

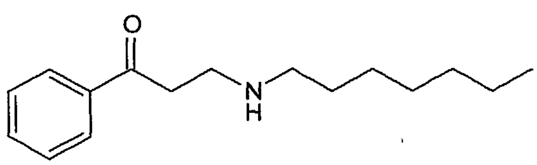

(IIc)

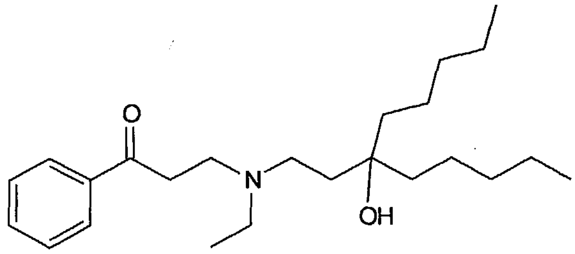

(IId)

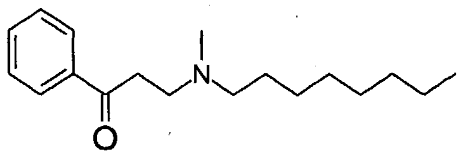

(IIe)

Ces composés peuvent généralement être préparés selon le schéma réactionnel suivant :

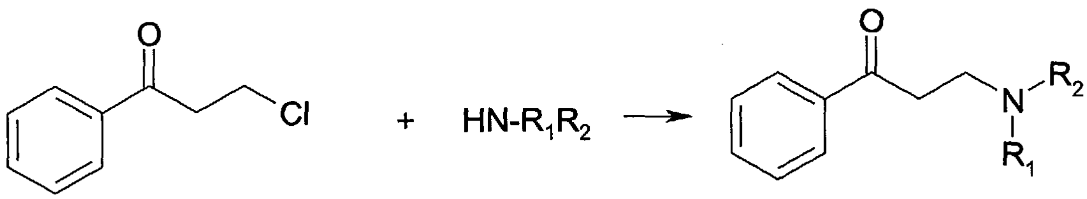

par substitution nucléophile, dans le méthanol à température ambiante, sous agitation magnétique. Le produit obtenu peut être traité et purifié sur silice.

Dans le cas du composé de formule (IId), l'amine de départ n'étant pas commerciale, on peut utiliser le procédé de préparation décrit à l'Exemple 1 ci-après.

La présente invention a encore pour objet une seconde sous-famille de nouveaux composés, inclus dans la formule générale (I) et répondant à la formule (III) suivante :

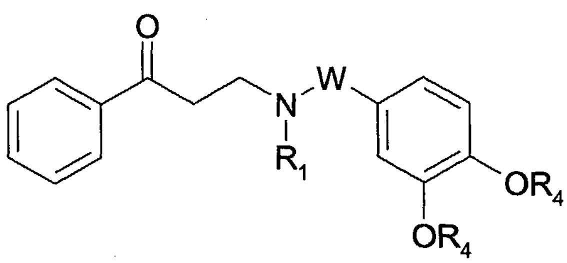

(III)

dans laquelle :

$R_1$ désigne un atome d'hydrogène ou un groupe méthyle,
W désigne une chaîne linéaire alkylène en $C_1$-$C_5$, pouvant être substituée par un groupe oxo ou un groupe hydroxyle, étant entendu que le composé de formule (III) ne comprend pas de fonction amide,
$R_4$ identiques ou différents désignent un atome d'hydrogène ou un groupe méthyle, ainsi que leurs sels d'addition

avec un acide.

L'acide utilisé pour salifier les composés de formule (III) peut être tout acide physiologiquement acceptable, tel que défini précédemment.

Des exemples de composés de formule (III) répondent aux formules (IIIa) et (IIIb) suivantes :

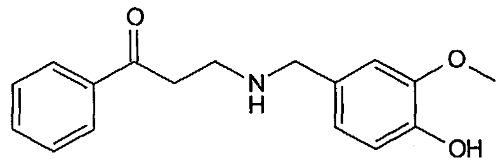

(IIIa)

HCl

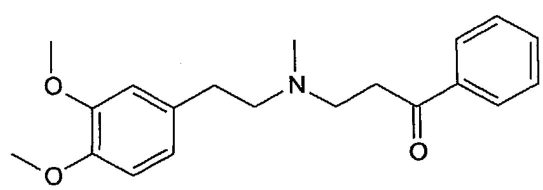

(IIIb)

Ces composés peuvent être préparés selon un procédé analogue au procédé de préparation des composés de formule (II) ci-dessus.

La présente invention a aussi pour objet une composition, en particulier adaptée à une application topique sur la peau, comprenant, dans un milieu physiologiquement acceptable, au moins un composé répondant à la formule (II) ou (III) ci-dessus, en particulier au moins l'un des composés (IIa) à (IId), (IIIa) et (IIIb).

La quantité de composé(s) de formule (I) -et donc de composés de formules (II) et (III)- utilisable selon l'invention est bien entendu fonction de l'effet recherché et peut donc varier dans une large mesure.

Pour donner un ordre de grandeur, on peut utiliser ces composés en une quantité représentant de 0,01 % à 10% du poids total de la composition, préférentiellement en une quantité représentant de 0,05% à 5% du poids total de la composition, plus préférentiellement en une quantité représentant de 0,1% à 2% du poids total de la composition.

La composition selon l'invention est adaptée à une application topique sur la peau et elle contient donc un milieu physiologiquement acceptable, c'est-à-dire compatible avec la peau et éventuellement avec ses phanères (cils, ongles, cheveux) et/ou les muqueuses. Ce milieu est avantageusement cosmétiquement acceptable, c'est-à-dire qu'il n'entraîne pas de démangeaisons, de picotements ou de rougeurs susceptibles de détourner l'utilisateur de la composition, et qu'il présente un aspect, une odeur et un toucher agréables.

Cette composition peut se présenter sous toutes les formes galéniques normalement utilisées dans le domaine cosmétique , et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les

conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des composés de formule (II) ou (III) selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine considéré. L'émulsionnant et le co-émulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et co-émulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, il sera avantageux d'introduire dans la composition utilisée selon l'invention au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents dermo-décontractants ; les agents tenseurs ; les agents anti-pollution et/ou anti-radicalaire ; les agents agissant sur la microcirculation ; les agents agissant sur le métabolisme énergétique des cellules ; et leurs mélanges.

Des exemples de tels composés additionnels sont donnés ci-dessous.

1. Agents desquamants

Par "agent desquamant", on entend tout composé capable d'agir :

- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol ;

- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

2. Agent hydratant

Par "agent hydratant", on entend :

- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide

ursolique, la vaseline et la lanoline ;

- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;

- soit un composé activant les glandes sébacées tel que la DHEA et ses dérivés, et la vitamine D et ses dérivés.

3. Agent dépigmentant ou pro-pigmentant

Les agents dépigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; l'acide ascorbique et ses dérivés, notamment le glucoside d'ascorbyle ; et les extraits de plantes, en particulier de réglisse, de mûrier et de scutellaire, sans que cette liste soit limitative.
Comme agent pro-pigmentant, on peut citer l'extrait de pimprenelle (Sanguisorba officinalis) commercialisé par la société MARUZEN et les extraits de chrysanthème (Chrysanthemum morifolium).

4. Agent anti-glycation

Par "agent anti-glycation", on entend un composé prévenant et/ou diminuant la glycation des protéines de la peau, en particulier des protéines du derme telles que le collagène.
Des exemples d'agents anti-glycation sont les extraits végétaux de la famille des Ericaceae, tels qu'un extrait de myrtille *(Vaccinium angustifolium)* ; l'ergothionéine et ses dérivés ; et les hydroxystilbènes et leurs dérivés, tels que le resvératrol et le 3,3', 5,5'-tétrahydroxystilbène.

5. Inhibiteur de NO-synthase

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

6. Agent stimulant la synthèse de macromolécules dermiques ou épidermiques et/ou empêchant leur dégradation

Parmi les actifs stimulant les macromolécules du derme ou empêchant leur dégradation, on peut citer ceux qui agissent :

- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines.

- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;

- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue

brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin® ;

- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;

- soit sur l'inhibition des métalloprotéinases (MMP) telles que plus particulièrement les MMP 1, 2, 3, 9. On peut citer : les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;

- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parelastyl® ; les héparinoïdes ; et les pseudodipeptides.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® ; et l'extrait de zooplancton Salina commercialisé par la société SEPORGA sous la dénomination commerciale GP4G®.

7. Agent stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine® ; et l'extrait de maïs commercialisé par la société SOLABIA sous la dénomination commerciale Phytovity®.

8. Agent dermo-décontractant

Outre le composé de formule (I) décrit précédemment, la composition selon l'invention peut comprendre d'autres agents dermo-décontractants, parmi lesquels on peut citer en particulier l'alvérine et ses sels, notamment le citrate d'alvérine, le gluconate de manganèse, les sapogénines telles que la diosgénine et les extraits naturels en contenant (tels que les extraits de Wild Yam), ainsi que l'hexapeptide argireline R commercialisé par la société LIPOTEC.

9. Agent tenseur

Par "agent tenseur", on entend un composé capable d'exercer une traction sur la peau, qui a pour effet d'estomper temporairement les irrégularités de la surface de la peau, telles que les rides et ridules.

Parmi les agents tenseurs utilisables dans la composition selon la présente invention, on peut citer notamment :

(1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,

(2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,

(3) les protéines et hydrolysats de protéines végétales, en particulier de maïs, de seigle, de froment, de sarrasin, de sésame, d'épautre, de pois, de fève, de lentille, de soja et de lupin,

(3) les silicates mixtes, notamment les phyllosilicates et en particulier les Laponites,

(4) les microparticules de cire, choisies par exemple parmi les cires de Carnauba, de Candelila ou d'Alfa,

(5) les particules colloïdales de charge inorganique ayant un diamètre moyen en nombre compris entre 0,1 et 100 nm, de préférence entre 3 et 30 nm, et choisies par exemple parmi : la silice, l'oxyde de cérium, l'oxyde de zirconium, l'alumine, le carbonate de calcium, le sulfate de baryum, le sulfate de calcium, l'oxyde de zinc et le dioxyde de titane.

10. Agent anti-pollution ou anti-radicalaire

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl®.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl®.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le coenzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

11. Les agents agissant sur la microcirculation

Les actifs agissant sur la microcirculation (vasoprotecteurs ou vasodilatateurs), se trouvent notamment parmi les flavonoïdes, les ruscogénines, les esculosides, l'escine extraite du marron d'Inde, les nicotinates, l'héperidine méthyl chalcone, les huiles essentielles de lavande ou de romarin, les extraits de *Ammi Visnaga.*

12. Les agents agissant sur le métabolisme énergétique des cellules

Par cette expression, on entend les actifs agissant sur le métabolisme énergétique cutané tel que, par exemple, et de façon non limitative, la synthèse d'ATP, ainsi que ceux qui interviennent sur la chaîne respiratoire de la cellule ou sur les réserves énergétiques. On peut citer à ce titre le Coenzyme Q10 (ubiquinone), le cytochrome C, la créatine ou encore la phosphocréatine.

Comme indiqué précédemment, la composition selon l'invention peut également renfermer des filtres UVA et/ou UVB, sous forme de composés organiques ou inorganiques, ces derniers étant éventuellement enrobés pour les rendre hydrophobes.

Les filtres organiques peuvent notamment être choisis parmi : les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-3 (Oxybenzone), ou la Benzophénone-4 (Uvinul MS40 disponible chez B.A.S.F.) ; les benzylidènes-camphres, en particulier le 3-benzylidène-camphre, l'acide benzylidènecampho-sulfonique, le benzalkoniumméthosulfate de camphre, le polyacrylamidométhylbenzylidène camphre, l'acide téréphthalylidène di-camphre sulfonique, et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.) ; les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane.

Les filtres inorganiques sont de préférence constitués d'oxyde de zinc et/ou de dioxyde de titane, de préférence de taille nanométrique, éventuellement enrobés d'alumine et/ou d'acide stéarique.

La composition selon l'invention est avantageusement destinée à être appliquée sur les zones du visage ou du front marquées par des rides et ridules d'expression, et/ou sur les personnes présentant des rides et ridules d'expression.

Les rides et ridules concernées sont de préférence celles disposées radialement autour de la bouche et/ou des yeux, en particulier les rides de la patte d'oie, et/ou situées au niveau du front, en particulier la ride dite du lion, située au niveau de la glabelle, dans l'espace inter-sourcillier, et/ou disposées horizontalement sur le front.

L'invention sera maintenant illustrée par les exemples non limitatifs suivants. Dans ces exemples, les quantités sont indiquées en pourcentage pondéral.

**EXEMPLES**

**Exemple 1 : Préparation de la 3-(éthyl-(3-hydroxy-3-pentyl-octyl)-amino) propiophénone**

Etape 1 : Synthèse de la 1-(Benzyl-éthyl-amino)-octan-3-one

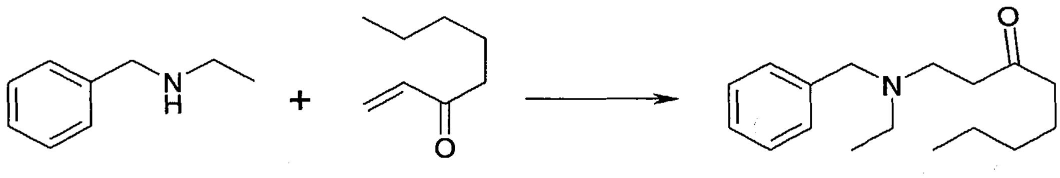

On fait réagir le 1-octène-3-one et l'éthyle benzylamine dans le méthanol à température ambiante. Le produit obtenu est traité et purifié sur colonne de silice. On récupère ainsi 1,4g de produit (rendement : 68%). Les analyses par spectrométrie de masse et RMN du proton sont conformes au produit attendu. On garde 300mg de cet intermédiaire pour test d'activité. Le reste est mis en réaction pour l'étape suivante.

Etape 2 : synthèse du 6-[2-(benzyl-éthyl-amino)-éthyl]-un décan-6-ol

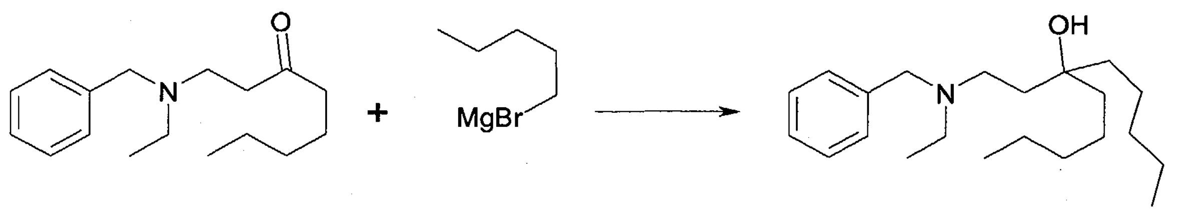

On fait réagir le bromure de pentylmagnésien sur le produit obtenu à l'étape 1, dans le THF anhydre, à température ambiante. Après traitement et purification sur silice, on obtient 750mg de produit (rendement : 50%).

Les analyses par spectrométrie de masse et RMN du proton sont conformes au produit attendu.

Etape 3 : synthèse du 6-(2-éthylamino-éthyl)-undécan-6-ol

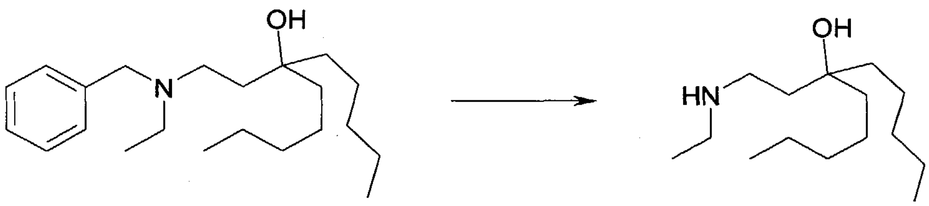

Le produit obtenu à l'étape 2 est débenzylé dans l'éthanol /eau sous pression d'hydrogène en présence de Pd/C. Après filtration sur célite en fin de réaction, on obtient 500 mg d'un solide quasiment monotache en chromatographie sur couche mince (rendement : 100%).

Les analyses par spectrométrie de masse et RMN du proton sont conformes au produit attendu.

Etape 4 : synthèse de la 3-[éthyl-(3-hydroxy-3-pentyl-octyl)-aminol]-1-phényl-propan-1-one

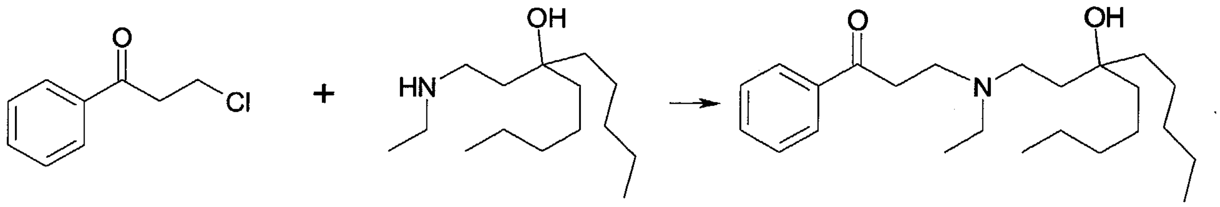

On fait réagir le produit issu de l'étape 3 sur la 3-chloropropiophénone dans le méthanol à température ambiante pendant 15h. Après traitement et purification sur silice, on récupère 300 mg de produit (rendement : 78%).
La spectrométrie de masse est conforme au produit attendu.

**Exemple 2 : Mise en évidence de l'effet dermo-décontractant des composés selon l'invention**

Le composé de formule (la) a été testé sur un modèle de derme équivalent qui permet de mettre en évidence la modulation de la contraction des fibroblastes insérés dans un gel de collagène fibrillaire. Ce modèle permet ainsi d'évaluer le potentiel relaxant de composés en mesurant le retard de contraction des dermes équivalents traités par rapport aux dermes équivalents témoins préparés dans les mêmes conditions. Le vérapamil est utilisé comme molécule de référence

1. Préparation des dermes équivalents

Des fibroblastes dermiques sont isolés à partir d'explants de peau humaine et cultivés en milieu MEM + additifs (sérum de veau foetal 10%, glutamine 1%, acides aminés non essentiels 1%, pyruvate de sodium 1%, Fungizone 1% et pénicilline /streptomycine 1%). Le collagène utilisé provient d'une solution commerciale. Il est extrait de queues de rat et conservé en milieu acide à +4 °C. Il est préalablement dialysé contre des bains successifs d'acide acétique de concentration décroissante.
Dans un tube à centrifuger de 50 ml conservé dans de la glace pilée sont ajoutés successivement les ingrédients suivants :

- MEM (1,76 x) avec ou sans composé à tester
- sérum de veau foetal
- NaOH (0,1 N)
- Acide acétique (1/1000)
- Collagène (3 mg/ml)
- 1,5 x $10^5$ fibroblastes / ml de MEM

Le mélange est homogénéisé avec précaution afin d'obtenir une répartition des cellules la plus homogène possible dans la matrice de collagène. Il est ensuite distribué dans chaque puits d'une boîte de culture 12 puits à raison de 2 ml/puits. La concentration cellulaire finale est de 3 x $10^4$ cellules / derme équivalent avec une concentration finale en collagène de 1 mg/ml.
Les boîtes de culture sont incubées à 37 °C / 5% $CO_2$ afin d'obtenir la polymérisation et la gélification du collagène. Les dermes équivalents ainsi traités adhèrent aux boîtes et les cellules sont soumises à des contraintes mécaniques qui potentialisent leurs propriétés contractiles. Les cellules ainsi cultivées en 3D se retrouvent dans des conditions proches de celles du derme. Après trois jours de culture dans ces conditions "attachées", les dermes équivalents sont détachés de leur support par agitation douce. Les cellules peuvent alors exercer leurs forces de traction sur les fibres de collagène conduisant à l'expulsion du milieu interstitiel et à la contraction du derme équivalent.

2. Traitement des dermes équivalents

Les dermes équivalents sont traités par les composés testés (composé la et vérapamil) dès J0, le jour de leur fabrication, en les introduisant dans la lattice collagénique et dans le milieu de culture. Le traitement est répété

par changement du milieu de culture à J3 après la libération des dermes équivalents, point de départ de la contraction. Le composé (la) est appliqué à la concentration finale de 1 µM et le vérapamil, utilisé comme molécule de référence, à 10 µM. Les dermes équivalents témoins sont mis en présence du véhicule ayant servi à la dilution du composé (la) et du vérapamil ,à savoir le DMSO.

Les résultats sont la moyenne de deux expériences indépendantes, chaque point expérimental étant réalisé en triplicate.

3. Mesure de la contraction

L'évaluation de la contraction spontanée des dermes équivalents traités (avec le composé la ou le vérapamil) et témoin (sans composé ajouté) est réalisée en mesurant leur surface, à différents temps après le début de la contraction spontanée.

Pour cela, une image numérique est acquise pour chaque derme équivalent traité ou non traité au moyen d'une caméra (Caméra CCD -Iris Sony DXC -107P) et sur chacune d'elles, les petit (D1 ) et grand (D2) diamètres du derme équivalent sont mesurés informatiquement. La surface est calculée selon la formule de calcul d'une ellipse : $S = (D1 \times D2 \times \pi) / 4$. La surface d'un puits d'une boîte de 12 puits (4 $cm^2$) est considérée comme la surface du derme équivalent à T0. La pourcentage de contraction est calculée selon la formule suivante où Si représente la surface du derme équivalent à Ti :

$$\% \text{ de contraction} = (4 - Si)/4 \times 100.$$

4. Résultats

Le composé de formule (la) testé à 1 µM entraîne une différence du taux de contraction de 25% après 4 jours de contact avec les fibroblastes, par rapport à celui mesuré pour les dermes équivalents témoins. A titre de comparaison, le vérapamil testé dans les mêmes conditions à 10 µM entraîne une différence du taux de contraction de 32%.

Il a par ailleurs été vérifié que cet effet du composé (la) ne résultait pas d'une éventuelle cytotoxicité.

En outre, l'alvérine testée dans les mêmes conditions ne présente pas d'activité dans ce test.

Ce résultat traduit l'activité dermo-décontractante ou dermo-relaxante des composés selon l'invention vis-à-vis des fibroblastes humains normaux, qui est comparable à celle du vérapamil.

Ainsi, les composés selon l'invention sont des agents dermo-décontractants susceptibles d'être utilisés dans le lissage des rides et ridules d'expression.

**Exemple 3 :Mise en évidence de l'effet inhibiteur calcique des composés selon l'invention**

Pour qu'une substance soit reconnue comme un inhibiteur des canaux calcium, elle doit pouvoir diminuer la concentration intracellulaire en calcium ou diminuer la liaison du calcium aux protéines intracellulaires comme par exemple la calmoduline, tel que cela est notamment décrit par Galizzi, J. P. et al., J. Biol. Chem. 1987, 262 p 6947 ; par Y. Okamiya et *al*., Eur. J. Pharmacol. 1991, 205, p 49 ; par J. A. Wagner et *al.,* J. Neurosci. 1988, 8, p 3354 ; par H. R. Lee et a*l*., Life Sci. 1984, 35, p 721; par Schoemaker H. et Lauger S. Eur. J. Pharmacol. 1985, 111, p 273 ou encore I. J. Reynolds et *al*., J. Pharmacol. Exp. Ther. 1986, 237, p 731.

Selon le protocole indiqué dans ces documents, le demandeur a déterminé l'$IC_{50ca2+}$ d'inhibition de flux calcique des composés (la), (IIa) à (IId), (IIIa) et (IIIb), en comparaison avec le vérapamil. Les résultats sont donnés dans le tableau ci-après. $IC_{50Ca2+}$ représente la concentration inhibitrice de 50 % de libération de $Ca^{2+}$.

| ***Composés testés*** | ***$IC_{50Ca2+}$ en µM*** | |
|---|---|---|
| | Sur cellules issues de fibroblastes de la peau HDFa | Sur cellules nerveuses SK-N-SH |
| alvérine | ND* | 30 |
| Composé (la) | ND | 36 |
| Composé (IIa) | 41 | 29 |
| Composé (IIb) | 31 | 26 |
| Composé (IIc) | 46 | 36 |

(suite)

| Composés testés | $IC_{50Ca2+}$ en μM | |
|---|---|---|
| | Sur cellules issues de fibroblastes de la peau HDFa | Sur cellules nerveuses SK-N-SH |
| Composé (IId) | 4 | 3 |
| Composé (IIIa) | 30 | 41 |
| Composé (IIIb) | 6 | 14 |

De ce tableau, il ressort que les amines auxquelles s'applique l'invention sont bien des inhibiteurs de canaux calcium.

De ces tests et de l'enseignement de la demande EP-1 053 745, on en déduit que les amines carbonylées de formules (I), (III) et (III) présentent une forte probabilité d'avoir un effet bénéfique sur les rides et en particulier les rides d'expression.

**Exemple 4 : Composition cosmétique**

Cette composition est préparée de manière classique pour l'homme du métier. Les quantités indiquées sont en pourcentages pondéraux.

| | |
|---|---|
| Composé de formule (Ia) | 1 % |
| Isostéarate de propylène glycol | 13 % |
| Polyéthylène glycol (8 OE) | 5 % |
| Propylène glycol | 3 % |
| Pentylène glycol | 3 % |
| Stéarate de glycéryle et stéarate de polyéthylène glycol (100 OE) | 5 % |
| Mono-stéarate de sorbitane oxyéthyléné (20 OE) | 0,5 % |
| Alcool cétylique oxyéthyléné (20 OE) oxypropyléné (5 OP) | 1 % |
| Gélifiants | 0,5 % |
| Benzoates d'alkyle en $C_{12-15}$ | 4 % |
| Ethanol | 3 % |
| Hydroxyde de sodium | 0,12% |
| Conservateurs | 0,7 % |
| Eau | qsp 100 % |

Ce fluide est destiné à être utilisé en applications mono- ou biquotidiennes sur le visage et le front pour atténuer les rides et ridules d'expression.

**Revendications**

1. Procédé de traitement cosmétique d'une peau ridée, comprenant l'application topique sur ladite peau d'une composition, adaptée à une application topique sur la peau, comprenant, dans un milieu physiologiquement acceptable, au moins un composé de formule (I) :

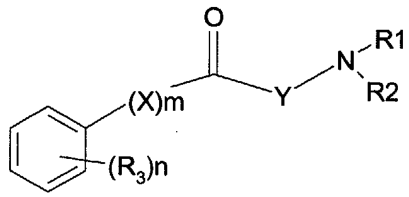

(I)

dans laquelle :

$R_1$ désigne un atome d'hydrogène ou un groupe alkyle linéaire ou ramifié, saturé ou insaturé, en $C_1$-$C_8$,
$R_2$ désigne :

- un groupe alkyle linéaire, ramifié ou cyclique, saturé ou insaturé, en $C_1$-$C_{20}$, éventuellement substitué par un groupe =O, et/ou par un plusieurs groupes -CN, -OR, -SR, -NRR', -COR, -COOR, -CONRR', -NR-CO-R', -NR-CO-NR'R", -$CF_3$ et/ou par un ou plusieurs atomes d'halogène et/ou par un groupe phényle éventuellement substitué par un groupe alkyle en $C_1$-$C_4$, un groupe alcoxy en $C_1$-$C_4$, un groupe hydroxy, un atome d'halogène et/ou un groupe $CF_3$,
- un groupe aryle éventuellement substitué par au moins un groupe choisi parmi : un groupe alkyle en $C_1$-$C_4$, un groupe alcoxy en $C_1$-$C_4$, un groupe hydroxy, un atome d'halogène ou un groupe $CF_3$,, ou
- un hétérocycle,

où R, R' et R" désignent indépendamment un atome d'hydrogène ou un groupe alkyle en $C_1$-$C_4$, linéaire ou ramifié, ou un groupe aryle,
$R_3$ désigne un groupe alkyle linéaire, ramifié ou cyclique, saturé ou insaturé, en $C_1$-$C_{12}$, ou un groupe aryle, éventuellement substitués par un groupe hydroxy, un groupe alcoxy en C1-C4 ou un atome d'hydrogène, un groupe -CN, -OR, -SR, -NRR', -COR, -COOR, -CONRR', -NR-CO-R', -NR-CO-NR'R" ou -$CF_3$ ou un atome d'halogène,
où R, R' et R" ont la signification indiquée précédemment,
X est un groupe alkylène linéaire en $C_1$-$C_9$, saturé ou insaturé, éventuellement substitué,
Y est un groupe alkylène linéaire en $C_1$-$C_{10}$, saturé ou insaturé, éventuellement substitué,
les substituants de X et Y étant indépendamment choisis parmi les groupements -OR, -SR, -NRR', -COR, -COOR, -CO-NRR', -NR-CO-R'R" et $CF_3$ où R, R' et R" ont la signification donnée ci-dessus,
m est 0 ou 1,
n est compris entre 0 et 3,

ou son sel d'addition avec un acide.

**2.** Procédé selon la revendication 1, **caractérisé en ce que** le sel du composé de formule (I) est obtenu par addition avec un acide inorganique choisi parmi les acides chlorhydrique, sulfurique, nitrique et phosphorique.

**3.** Procédé selon la revendication 1, **caractérisé en ce que** le sel du composé de formule (I) est obtenu par addition avec un acide organique choisi parmi les acides succinique, fumarique, lactique, glycolique, citrique et tartrique.

**4.** Procédé selon l'une quelconque des revendications 1 à 3, **caractérisé en ce que** m = 0 dans la formule (I).

**5.** Procédé selon l'une quelconque des revendications 1 à 4, **caractérisé** en ce n = 0 dans la formule (I).

**6.** Procédé selon l'une quelconque des revendications 1 à 5, **caractérisé en ce que** Y est un groupe alkylène linéaire en $C_1$-$C_3$ dans la formule (I).

**7.** Procédé selon l'une quelconque des revendications 1 à 6, **caractérisé en ce que** $R_1$ est un groupe alkyle en $C_1$-$C_3$ dans la formule (I).

**8.** Procédé selon l'une quelconque des revendications 1 à 7, **caractérisé en ce que** $R_2$ est un groupe alkyle en

$C_1$-$C_3$ substitué par un groupe arylcarbonyle dans la formule (I).

**9.** Procédé selon l'une quelconque des revendications 1 à 8, **caractérisé en ce que** ledit composé de formule (I) répond à la formule (Ia) :

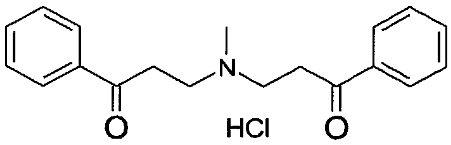

(Ia)

**10.** Procédé selon l'une quelconque des revendications 1 à 9, **caractérisé en ce que** ledit composé de formule (I) représente de 0,1 à 2% du poids total de la composition.

**11.** Procédé selon l'une quelconque des revendications 1 à 10, **caractérisée en ce que** ladite composition renferme en outre au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents dermo-décontractants ; les agents tenseurs ; les agents anti-pollution et/ou anti-radicalaire ; les agents agissant sur la microcirculation ; les agents agissant sur le métabolisme énergétique des cellules ; et leurs mélanges.

**12.** Procédé selon l'une quelconque des revendications 1 à 11, **caractérisé en ce que** ladite composition est appliquée sur les zones du visage ou du front marquées par des rides et ridules d'expression et/ou sur les personnes présentant des rides et ridules d'expression.

**13.** Procédé selon l'une quelconque des revendications 1 à 12, **caractérisé en ce que** ladite composition est appliquée sur les rides et ridules disposées radialement autour de la bouche et/ou des yeux et/ou horizontalement sur le front et/ou situées dans l'espace inter-sourcillier.

**14.** Utilisation cosmétique d'au moins un composé de formule (I) tel que défini dans l'une quelconque des revendications 1 à 9, dans une composition adaptée à une application topique sur la peau, comme agent destiné à lisser les rides et ridules.

**15.** Utilisation selon la revendication 14, **caractérisée en ce que** lesdites rides et ridules sont des rides et ridules d'expression.

**16.** Composés répondant à la formule (II) :

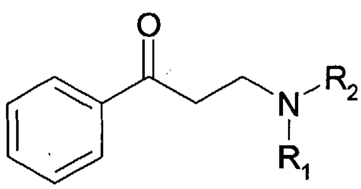

(II)

dans laquelle :

$R_1$ est un atome d'hydrogène ou un groupe méthyle ou éthyle,
$R_2$ est un radical alkyle linéaire ou ramifié en $C_6$-$C_{20}$ pouvant être substitué par un groupe oxo ou un groupe hydroxyle, et leurs sels d'addition avec un acide.

**17.** Composés selon la revendication 16, **caractérisés en ce qu'**ils répondent à l'une des formules (IIa) à (IId)

suivantes :

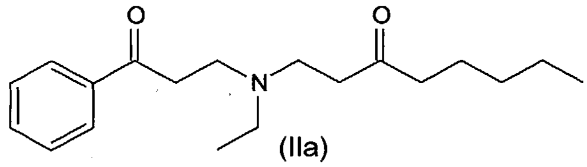

(IIa)

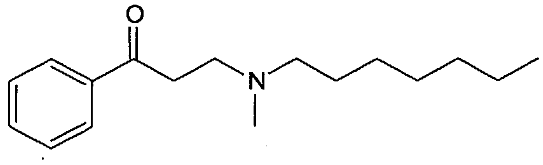

(IIb)

HCl

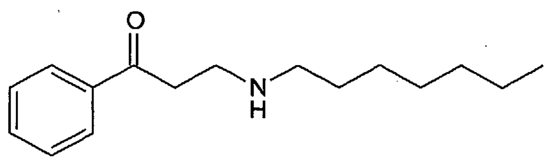

(IIc)

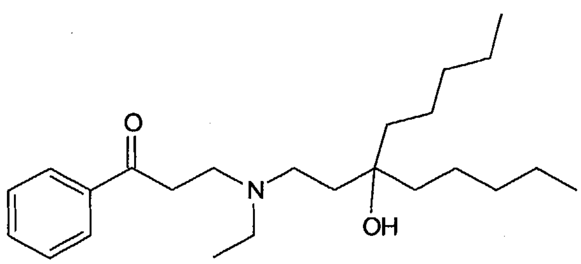

(IId)

**18.** Composés répondant à la formule (III) :

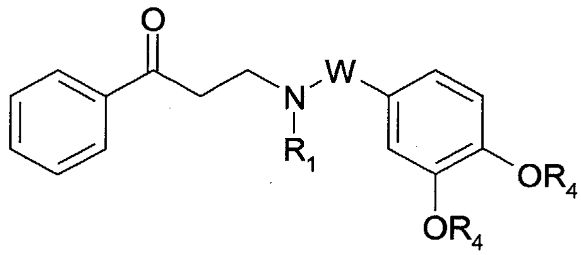

(III)

dans laquelle :

$R_1$ désigne un atome d'hydrogène ou un groupe méthyle,
W désigne une chaîne linéaire alkylène en $C_1$-$C_5$, pouvant être substituée par un groupe oxo ou un groupe hydroxyle, étant entendu que le composé de formule (III) ne comprend pas de fonction amide,
$R_4$ identiques ou différents désignent un atome d'hydrogène ou un groupe méthyle, ainsi que leurs sels d'addition avec un acide.

**19.** Composés selon la revendication 18, **caractérisés en ce qu'**ils répondent à l'une des formules (IIIa) et (IIIb) suivantes :

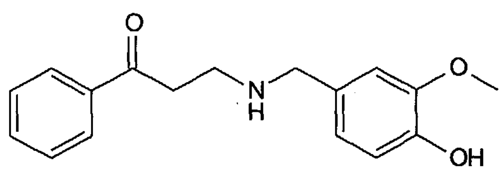

(IIIa)

HCl

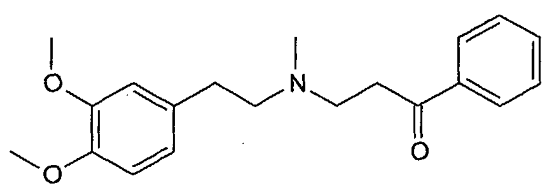

(IIIb)

**20.** Composition, en particulier destinée à une application topique sur la peau, comprenant au moins l'un des composés selon l'une quelconque des revendications 16 à 19 dans un milieu physiologiquement acceptable.

Office européen des brevets

# RAPPORT DE RECHERCHE EUROPEENNE

Numéro de la demande
EP 03 29 2265

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 3 644 525 A (THIELE KURT) 22 février 1972 (1972-02-22) * colonne 2, ligne 10 - ligne 40; revendication 1; exemple 6 * | 18,20 | A61K7/48 |
| X | GB 1 040 722 A (DEGUSSA) 1 septembre 1966 (1966-09-01) * exemple 20 * | 18 | |
| X | DATABASE CROSSFIRE BEILSTEIN [en ligne] Beilstein Institut zur Förderung der Chemischen Wissenschaften, Frankfurt am Main, DE; Database accession no. 3091762 (BRN) XP002268042 * abrégé * & COLLECT. CZECH. CHEM. COMMUN. , vol. 33, 1968, page 3227 | 16 | |
| X | DATABASE CROSSFIRE BEILSTEIN [en ligne] Beilstein Institut zur Förderung der Chemischen Wissenschaften, Frankfurt am Main, DE; Database accession no. 5822517 (BRN) XP002268043 * abrégé * & EUR. J. MED. CHEM. CHIM. THER., vol. 27, no. 6, 1992, pages 595-610, | 16 | DOMAINES TECHNIQUES RECHERCHES (Int.Cl.7): A61K |
| D,A | FR 2 798 590 A (OREAL) 23 mars 2001 (2001-03-23) * page 3, ligne 18 - ligne 26; revendications 1,6,9 * | 1-15 | |
| D,A | US 6 372 772 B1 (KIRKPATRICK D LYNN ET AL) 16 avril 2002 (2002-04-16) * colonne 11, ligne 52 - ligne 65; revendication 1; exemples 382007,382000 * | 1,14,20 | |

-/--

Le présent rapport a été établi pour toutes les revendications

| Lieu de la recherche | Date d'achèvement de la recherche | Examinateur |
|---|---|---|
| LA HAYE | 26 janvier 2004 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 03 29 2265

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | DATABASE CA [en ligne] CHEMICAL ABSTRACTS SERVICE, COLUMBUS, OHIO, US; MATSUDA, HIROSHI: "Studies on the change in the shape of the melanophores in the frog-- skin. II. The influence of adrenaline and drugs related to adrenaline and histamine on the extension of melanophores produce by means of posterior pituitary lobe extract" retrieved from STN Database accession no. 29:57100 XP002268044 * abrégé * & FOLIA PHARMACOL. JAPON. (1935), 20, 90-116(BREVIARIA 54), --- | 1,14,20 | |
| D,A | HALISE INCI GUL ET AL: "Antifungal evaluation of bis Mannich bases derived from acetophenones and their corresponding piperidinols and stability studies" BIOLOGICAL AND PHARMACEUTICAL BULLETIN, vol. 25, no. 10, 1 octobre 2002 (2002-10-01), pages 1307-1310, XP001149166 * abrégé; exemple B1 * --- -/-- | 1,14,20 | DOMAINES TECHNIQUES RECHERCHES (Int.Cl.7) |

Le présent rapport a été établi pour toutes les revendications

| Lieu de la recherche | Date d'achèvement de la recherche | Examinateur |
|---|---|---|
| LA HAYE | 26 janvier 2004 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 03 29 2265

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,A | DATABASE CA [en ligne] CHEMICAL ABSTRACTS SERVICE, COLUMBUS, OHIO, US; CHAWLA, H. P. S. ET AL: "Agents acting on the central nervous system. XII. 3-Tert-aminopropiophenones as central muscle relaxants and diuretics" retrieved from STN Database accession no. 72:132240 XP002244926 * composé du RN:28169-03-1* * abrégé * & JOURNAL OF MEDICINAL CHEMISTRY (1970), 13(3), 480-8, --- | 1,14,20 | |
| A | WO 95 18092 A (MARUHO KK ;MATSUI TAKEAKI (JP); YAMAZAKI HIROFUMI (JP); HASHINAGA) 6 juillet 1995 (1995-07-06) * abrégé * * page 59 * --- | 1,14,20 | |
| | | | DOMAINES TECHNIQUES RECHERCHES (Int.Cl.7) |
| A | D. FLAMMIA ET AL: "Lobeline: Structure-Affinity investigation of nicotinic acetylcholinergic receptor binding" JOURNAL OF MEDICINAL CHEMISTRY, vol. 42, 1999, pages 3726-3731, XP001159157 * page 3729; exemple 13 * --- | 1,14,20 | |
| D,A | EP 1 090 630 A (SOKEN KK) 11 avril 2001 (2001-04-11) * revendication 1 * --- | 1,14,20 | |

-/--

Le présent rapport a été établi pour toutes les revendications

| Lieu de la recherche | Date d'achèvement de la recherche | Examinateur |
|---|---|---|
| LA HAYE | 26 janvier 2004 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 03 29 2265

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 0 416 564 A (KAO CORP)<br>13 mars 1991 (1991-03-13)<br>* page 11; revendication 1; exemples IK,IL *<br>----- | 1,14,20 | |
| | | | DOMAINES TECHNIQUES RECHERCHES (Int.Cl.7) |

Le présent rapport a été établi pour toutes les revendications

| Lieu de la recherche | Date d'achèvement de la recherche | Examinateur |
|---|---|---|
| LA HAYE | 26 janvier 2004 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO. EP 03 29 2265

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

26-01-2004

| Document brevet cité au rapport de recherche | Date de publication | Membre(s) de la famille de brevet(s) | Date de publication |
|---|---|---|---|
| US 3644525 A | 22-02-1972 | AUCUN | |
| GB 1040722 A | 01-09-1966 | BE 630296 A | |
| | | CH 473089 A | 31-05-1969 |
| | | CH 499493 A | 30-11-1970 |
| | | DE 1493574 A1 | 06-03-1969 |
| | | DE 1493576 A1 | 27-03-1969 |
| | | DE 1493579 B1 | 26-08-1971 |
| | | DE 1493580 A1 | 27-03-1969 |
| | | DE 1793635 A1 | 02-03-1972 |
| | | DE 1250450 B | |
| | | DK 107685 C | 26-06-1967 |
| | | DK 107686 C | 26-06-1967 |
| | | DK 107490 C | 05-06-1967 |
| | | FI 42098 B | 02-02-1970 |
| | | FI 42096 B | 02-02-1970 |
| | | FR 1353729 A | 28-02-1964 |
| | | GB 1040723 A | 01-09-1966 |
| | | GB 1040724 A | 01-09-1966 |
| | | GB 1094461 A | 13-12-1967 |
| | | IN 142384 A1 | 02-07-1977 |
| | | IT 1061751 B | 30-04-1983 |
| | | SE 304278 B | 23-09-1968 |
| | | SE 321241 B | 02-03-1970 |
| | | SE 304017 B | 16-09-1968 |
| | | US 3225095 A | 21-12-1965 |
| FR 2798590 A | 23-03-2001 | FR 2798590 A1 | 23-03-2001 |
| | | CA 2319842 A1 | 21-03-2001 |
| | | EP 1088548 A2 | 04-04-2001 |
| | | JP 2001114676 A | 24-04-2001 |
| | | US 6335368 B1 | 01-01-2002 |
| US 6372772 B1 | 16-04-2002 | AU 5463099 A | 21-02-2000 |
| | | CA 2339233 A1 | 10-02-2000 |
| | | WO 0006088 A2 | 10-02-2000 |
| WO 9518092 A | 06-07-1995 | WO 9518092 A1 | 06-07-1995 |
| EP 1090630 A | 11-04-2001 | AU 2747199 A | 27-09-1999 |
| | | CA 2323451 A1 | 16-09-1999 |
| | | EP 1090630 A1 | 11-04-2001 |
| | | CN 1292682 T | 25-04-2001 |
| | | WO 9945900 A1 | 16-09-1999 |
| EP 0416564 A | 13-03-1991 | JP 1905538 C | 24-02-1995 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 03 29 2265

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

26-01-2004

| Document brevet cité au rapport de recherche | Date de publication | Membre(s) de la famille de brevet(s) | Date de publication |
|---|---|---|---|
| EP 0416564 A | | JP 3220153 A | 27-09-1991 |
| | | JP 6035416 B | 11-05-1994 |
| | | EP 0416564 A2 | 13-03-1991 |
| | | US 5146002 A | 08-09-1992 |
| | | US 5191121 A | 02-03-1993 |
| | | JP 3188041 A | 16-08-1991 |

EPO FORM P0460

Pour tout renseignement concernant cette annexe : voir Journal Officiel de l'Office européen des brevets, No.12/82